Europäisches Patentamt

(19) European Patent Office

Office européen des brevets

(11) EP 0 639 069 B1

(12) **FASCICULE DE BREVET EUROPEEN**

(45) Date de publication et mention
de la délivrance du brevet:
**29.10.1997 Bulletin 1997/44**

(51) Int. Cl.⁶: **A61K 9/00**, A61K 9/20,
A61K 9/28

(21) Numéro de dépôt: 93910086.3

(22) Date de dépôt: 05.05.1993

(86) Numéro de dépôt international:
**PCT/FR93/00433**

(87) Numéro de publication internationale:
**WO 93/21901 (11.11.1993 Gazette 1993/27)**

(54) **FORME GALENIQUE POUR ADMINISTRATION OCULAIRE ET PROCEDE DE PREPARATION**

OKULAERDARREICHUNGSFORM UND VERFAHREN ZU IHRER HERSTELLUNG

GALENIC FORM FOR OCULAR ADMINISTRATION AND PROCESS FOR THE PREPARATION OF SAME

(84) Etats contractants désignés:
**BE CH DE ES GB IT LI NL**

(30) Priorité: **05.05.1992 FR 9205514**

(43) Date de publication de la demande:
**22.02.1995 Bulletin 1995/08**

(73) Titulaires:
• **Aiache, Jean-Marc**
  **F-63000 Clermont-Ferrand (FR)**
• **SERPIN, Gilbert**
  **F-63000 Clermont-Ferrand (FR)**

(72) Inventeurs:
• **Aiache, Jean-Marc**
  **F-63000 Clermont-Ferrand (FR)**

• **SERPIN, Gilbert**
  **F-63000 Clermont-Ferrand (FR)**

(74) Mandataire: **Warcoin, Jacques**
  **Cabinet Régimbeau,**
  **26, avenue Kléber**
  **75116 Paris (FR)**

(56) Documents cités:
  **FR-A- 2 140 114        US-A- 3 630 200**

Remarques:
  Le dossier contient des informations techniques
  présentées postérieurement au dépôt de la
  demande et ne figurant pas dans le présent
  fascicule.

**Description**

La présente invention concerne des formes galéniques destinées à l'administration oculaire de principes actifs que l'on souhaite délivrer de façon plus ou moins prolongée, ainsi qu'un procédé permettant leur préparation.

La pénétration des principes actifs dans l'oeil se fait essentiellement par la conjontive, qui est très vascularisée. La pénétration par la cornée est très discutée ; elle serait très sélective et influencée par de nombreux facteurs. L'épithélium jouerait le rôle de barrière.

La sécrétion et le drainage du liquide lacrymal entraînent une rapide élimination de toutes les formes médicamenteuses conventionnelles appliquées à la surface de l'oeil. Pour les collyres, 20 % de la goutte instillée peuvent être retenus par l'oeil, cinq minutes après administration seulement 8 % des 20 % restent dans le cul-de-sac conjonctival. Ceci est dû au renouvellement du liquide lacrymal pouvant être accru du fait de l'irritation causée par la goutte. De ce faible temps de contact, ou encore des liaisons aux protéines, il en résulte qu'il n'y a qu'une petite fraction qui pénètre dans l'oeil. Les moyens conventionnels tels que l'augmentation de la viscosité des collyres, l'adjonction de tensio-actifs, les solutions huileuses ou les pommades sont peu efficaces.

En plus de la prolongation d'action, les nouvelles technologies développées ces dernières années cherchent à apporter d'autres avantages tels qu'une concentration régulière de principes actifs dans le liquide lacrymal, pour une réponse pharmacologique plus soutenue, une diminution des effets secondaires et une posologie moins astreignante, ce qui devrait améliorer l'observance du patient.

C'est dans ce but que les implants extra-oculaires insolubles du type Ocusert® sont apparus en 1973 aux Etats-Unis. Ils se présentent sous la forme de deux membranes serties, insolubles, semi-perméables renfermant la solution de principes actifs médicamenteux qui en quelques jours se relarguent lentement au niveau des culs-de-sac conjonctivaux. Cependant, ce type de produit entraîne de nombreux problèmes de tolérance, qui limitent leur utilisation.

Des implants extra-oculaires ont alors été mis au point. Ils font l'objet d'une monographie Pro-Pharmacopea en avril-mai 1988 (N° 500) sous le nom d'insert ophtalmiques. Hitoschi Ozawa (Biomaterials, vol. 4, juillet 1983) a étudié l'intérêt d'inserts oculaires imprégnés avec des antibiotiques (dont l'érythromycine) dans le traitement du trachome chez les lapins et leur comportement in vitro. Malgré leur activité satisfaisante in vivo, les inserts solubles ou biodégradables ont cependant l'inconvénient de pouvoir laisser des débris et d'entraîner des troubles de la vision résultant de la dissolution du polymère. C'est pourquoi leur utilisation dans le cadre d'un acte chirurgical semble à écarter.

Le brevet US- 3 630 200 décrit des inserts oculaires flexibles comprenant un noyau interne qui est soit constitué de capsules creuses dans lequel le principe actif est encapsulé, soit obtenu par polymérisation d'un mélange de monomères constitutifs et du principe actif sous forme liquide.

FR- 2 140 114 décrit des compositions comprenant une ou plusieurs substances lipophiles ayant un point de fusion de 37 à 75° C et un alcool supérieur tensioactif, ces compositions pouvant être façonnées sous forme de dose unitaire d'administration, qui va ramollir à la température du corps.

L'usage d'une forme solide insoluble, pouvant être facilement retirée de l'oeil après avoir relargué les principes actifs serait préférable. En outre, pour certaines interventions chirurgicales de l'oeil, il est nécessaire de maintenir l'oeil en état de mydriase et de cycloplégie pendant plusieurs heures voire jours.

C'est pourquoi la présente invention à pour objet une forme galénique destinée à l'administration oculaire, caractérisé en ce qu'il s'agit d'un comprimé solide qui comporte, de l'intérieur vers l'extérieur :

- une matrice, constituée d'au moins un polymère, et renfermant au moins un principe actif,
- une première couche de glycérides.

Le ou les polymères constituant la matrice forment un réseau qui emprisonne les molécules de principe actif et les relarguent progressivement. Une partie des glycérides formant la première couche de revêtement peut être présente au sein de la matrice. Ils assurent un rôle de lubrifiant lors de la compression. Leur quantité est modulée de façon à ce qu'ils se répartissent à la surface du noyau et forment une couche régulière et homogène, d'épaisseur variable en fonction de la vitesse de libération du principe actif qui est recherchée.

Le noyau central de la forme galénique peut également contenir les excipients nécessaires à sa formulation et connus de l'homme du métier, tels que mouillant, liant, diluant appropriés.

Le(s) polymère(s) utilisé pour la matrice est notamment choisi dans le groupe comprenant polyéthylène, polymère acrylique, chlorure de polyvinyle, dérivés cellulosiques du type HPMC, alcool polyvinylique, Eudragit®.

Les Eudragit sont une famille de composés de type résine acrylique commercialisés par Röhm Pharma GmbH.

Par glycérides solides, on entend des glycérides se présentant soit en poudre, soit sous forme liquide adsorbée sur des supports. On utilise de préférence des glycérides naturels ou semi-synthétiques, en particulier des triglycérides. On peut citer par exemple le Compritol®, ou béhénate de glycérol.

Selon l'un des aspects de l'invention, la forme galénique comporte en outre une seconde couche, formant une membrane externe semi-perméable.

Cette membrane externe a une double fonction. D'une part, en jouant le rôle de barrière dialysante, elle permet un meilleur contrôle de la vitesse de relargage du ou des principes actifs présents dans la structure matricielle du noyau.

D'autre part, cet enrobage améliore la tolérance de la forme galénique in vivo et donc permet le maintien prolongé sans les effets secondaires d'irritation observés avec les inserts classiques.

La seconde couche est formée à partir d'un dérivé thermo-plastique de cellulose, par exemple d'éthylcellulose ou d'acétate de cellulose. Il appartient à l'homme du métier de choisir tout autre composé capable d'assurer les fonctions de membrane semi-perméable.

La forme galénique selon l'invention est un comprimé pouvant être placé dans le cul-de-sac conjonctival du patient, afin d'y libérer progressivement les principes actifs.

De manière avantageuse, ses dimensions sont comprises, pour la longueur entre environ 2,5 et 4 mm et pour le diamètre entre environ 1 et 3 mm.

De tels comprimés présentent des propriétés optimales en ce qui concerne la composition qualitative, la taille et la solidité des comprimés. Ces formes peuvent se placer et se retirer facilement du cul-de-sac conjonctival sans laisser de débris.

De préférence, les formes galéniques selon l'invention contiennent au moins un principe actif choisi dans le groupe comprenant les mydriatiques, les antibiotiques et les antiinflammatoires.

Selon l'un des aspects de l'invention, on prépare des comprimés de forme allongée, renfermant, dans la matrice centrale, deux mydriatiques, agissant selon un mécanisme différent, la néosynéphrine et la tropicamide.

La néosynéphrine, utilisée sous forme de son chlorhydrate, est un stimulant alpha-adrénergique utilisé comme vasoconstricteur décongestionnant et mydriatique.

La tropicamide est un parasympatholytique de la famille des antimuscariniques, qui bloque les récepteurs de l'acétylcholine, essentiellement ceux du sphincter irien et des muscles ciliaires ; elle est utilisée en clinique dans le traitement des uvéites afin d'obtenir une cycloplégie, notamment pour l'intervention sur le cristallin.

L'association de ces deux principes actifs permet d'observer une synergie. De tels comprimés, après stérilisation aux rayons gamma, peuvent être placés 1 à 2 heures dans le cul-de-sac conjonctival de patients en pré-opératoires puis retirés juste avant l'opération chirurgicale de l'oeil mis en condition.

De manière préférée, dans la forme galénique selon l'invention, le principe actif est présent à un taux compris entre 1 et 60 %, le polymère de la matrice est présent à un taux compris entre 25 et 70 %, et les glycérides sont présents à un taux de 8 à 40 %, par rapport au poids total du comprimé.

La présente invention a également pour objet un procédé de préparation d'une forme galénique présentant une ou plusieurs des caractéristiques définies ci-dessus, dans lequel on effectue les étapes suivantes :

a) mélange du ou des principes actifs avec le ou les polymères constitutifs de la matrice,
b) de manière facultative, granulation du mélange obtenu après mouillage et incorporation d'adjuvants,
c) addition des glycérides et mélange,
d) compression directe du mélange obtenu à l'issue de l'étape précédente.

Le mélange du ou des principes actifs avec les polymères et d'éventuels adjuvants doit être suffisament homogène pour que les glycérides se répartissent à l'intérieur et à l'extérieur du comprimé lors de la compression ; dans ce but, on procédera de manière facultative à une granulation, avant l'addition des glycérides.

Selon l'un de ses aspects, le procédé selon l'invention est caractérisé en ce que après l'étape (d), on effectue une étape d'enrobage du comprimé par un dérivé thermoplastique en solution dans un solvant organique.

Cette étape supplémentaire permet de former la seconde couche ou membrane dialysante.

L'enrobage peut notamment être effectué en turbine ou sous lit d'air fluidisé.

L'éthylcellulose est par exemple utilisée en solution hydro-alcoolique et le séchage est fait sous lit d'air fluidisé.

En vue de leur utilisation, ces comprimés sont ensuite stérilisés, de préférence par rayonnements gamma.

D'autres caractéristiques et avantages de la présente invention apparaîtront mieux à la lecture des exemples qui suivent, qui ne sont nullement destinés à en limiter la portée.

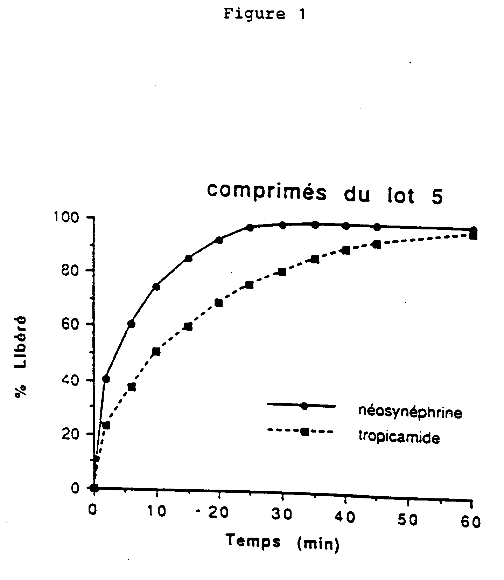

Dans ces exemples, on se référera à la figure 1, qui représente la courbe des pourcentages libérés de principe actif, cumulés, pour les comprimés de formule 5, en fonction du temps.

## EXEMPLE 1

Différentes formes galéniques selon l'invention sont préparées par la méthode suivante :

La néosynéphrine se présente sous forme de petits cristaux. Ces comprimés étant à destinée ophtalmique, il est nécessaire de disposer de poudres très fines. La néosynéphrine chlorhydrate est donc broyée préalablement, à l'inverse de la tropicamide dont la poudre est suffisamment fine. Les principes actifs sont alors tamisés sur un tamis de maille 0,400 mm ainsi que les excipients de la phase interne et externe.

Puis les principes actifs et les excipients sont pesés et mélangés au Turbula pendant dix minutes.

Le mouillage du mélange de poudre est réalisé au Kenwood suivi d'une granulation sur grille 1 mm. Les grains obtenus sont séchés à l'étuve ventilée, 4 heures à 45° C, avant d'être calibrés sur grille 0,630 mm, pour les formules : 1,2,3 et 0,315 mm pour les formules : 4,5,5' pour des raisons exposées par la suite. Les grains sont ensuite pesés et mélangés à la phase externe.

La dernière étape consiste en une compression directe sur une machine alternative.

**FORMULES**

**\* Formule 1**

|  | **Formule unitaire pour m = 25 mg** |
|---|---|
| Tropicamide : 2,5 % | 0,625 mg |
| Néosynéphrine : 50 % | 12,500 mg |
| Ethylcellulose N10 : 42,5 % | 10,625 mg |
| Compritol : 5 % | 1,250 mg |
| Eau distillée : Q.S. |  |
|  | 25,000 mg |

-Le compritol est utilisé en phase externe en tant que lubrifiant de compression directe.
- Une partie de l'éthylcellulose et l'eau sont utlisées pour le mouillage, le reste de l'éthylcellulose a un rôle de liant.

**\* Formule 2**

|  | **Formule unitaire pour m = 25 mg** |
|---|---|
| Tropicamide : 0,25 % | 0,0625 mg |
| Néosynéphrine : 50 % | 12,5000 mg |
| Eudragit RLPM : 41,15 % | 10,2875 mg |
| Eudragit NE30D : 3,6 % | 0,9000 mg |
| Compritol : 5 % | 1,2500 mg |
|  | 25,0000 mg |

-L'eudragit RLPM est utilisé ici en tant que liant et sert à faire la matrice.
- L'eudragit NE30D est employé comme liquide de mouillage.
- Le compritol sert en phase externe (lubrifiant).

**\* Formule 3**

|  | Formule unitaire pour<br>m = 25 mg |
|---|---|
| Tropicamide : 0,20 % | 0,050 mg |
| Néosynéphrine : 49,8 % | 12,450 mg |
| Eudragit RLPM : 38,4 % | 9,600 mg |
| Eudragit NE30D : 3,6 % | 0,900 mg |
| Compritol : 8 % | 2,000 mg |
|  | 25,000 mg |

* Formule 4

|  | Formule unitaire pour<br>m = 12,5 mg |
|---|---|
| Tropicamide : 2,5 % | 0,3125 mg |
| Néosynéphrine : 50 % | 6,2500 mg |
| Eudragit RLPM : 35,9 % | 4,4875 mg |
| Eudragit NE30D : 3,6 % | 0,4500 mg |
| Compritol : 8 % | 1,0000 mg |
|  | 12,5000 mg |

-Pour le premier lot de la formule 4, les grains ont été granulés sur grille 0,630 mm, mais les poinçons sont ici deux fois plus petits que pour les précédentes formules, la chambre de compression est réduite de moitié et les grains se sont révélés trop gros pour un bon remplissage de la matrice. Les grains ont donc été calibrés sur une grille de 0,315 mm, les grains ainsi réduits ont permis une bonne compression.

* Formule 5

|  | Formule unitaire pour<br>m = 11,2 mg |
|---|---|
| Tropicamide : 2,5 % | 0,2800 mg |
| Néosynéphrine : 48 % | 5,3760 mg |
| Eudragit RLPM : 35,9 % | 4,0208 mg |
| Eudragit NE30D : 3,6 % | 0,4032 mg |
| Compritol : 10 % | 1,1200 mg |
|  | 11,2000 mg |

Les poinçons utilisés sont les mêmes que pour la formule 4 et le grain est calibré sur une grille de maille 0,315 mm.

**\* Formule 5'**

C'est la même formule que la formule 5' mais les comprimés de 11,2 mg obtenus sont ensuite enrobés avec une solution hydroalcoolique à 10 % d'éthylcellulose.

La solution d'enrobage est préparée la veille et mise à agiter toute la nuit. Elle a la formule suivante :

- alcool à 95° : 90 ml
- éthylcellulose : 10 g

Les comprimés sont trempés un par un à l'aide d'une petite passoire, cinq fois successives, entre chaque trempage ils sont séchés posés sur du papier intissé à l'étuve à 50° C.

Le dosage de ces comprimés avant enrobage est le même que ceux de la formule 5.

## EXEMPLE 2

La cinétique de libération des principes actifs a été étudiée par la technique suivante.

On place le comprimé dans un tube en plastique fermé hermétiquement de capacité 20 ml et rempli de 20 ml d'une solution de NaCl à 0,9 %. Le tube est ensuite fixé au système d'agitation de l'appareil de dissolution **in vitro** des Bouteilles Tournantes (décrit dans le National Formulary XV) et agité à la vitesse constante de 20 tour/minute). L'ensemble est placé dans une étuve à 37° C. Le dixième du liquide (soit 2 ml) est alors prélevé à des temps déterminés et renouvelé par la même quantité de solvant neuf (NaCl 0,9 %). Les temps de prélèvement sont : 2,6,10,15,20,25,30,35,40,45,60 minutes. Les échantillons sont ensuite analysés par H.P.L.C.

Les pourcentages libérés **in vitro** cumulés sont décrits par une courbe avec le temps en abscisse et les pourcentages libérés en ordonnée.

Pour les comprimés, répondant à la formule 5, les pourcentages libérés cumulés **in vitro** de néosynéphrine par rapport au temps sont regroupés dans les tableaux 1 et 2, et la courbe obtenue représentée figure 1.

Tableau 1

| Temps (minute) | Comprimé n° 1 | Comprimé n° 2 | Comprimé n° 3 | Moyenne (%) | Ecart-type |
|---|---|---|---|---|---|
| 2 | 43,8 | 37,3 | 40,2 | 40,4 | 2,7 |
| 6 | 58,4 | 58,8 | 65,3 | 60,8 | 3,2 |
| 10 | 74,7 | 73,9 | 75,8 | 74,8 | 0,8 |
| 15 | 83,1 | 85,3 | 88,4 | 85,6 | 2,2 |
| 20 | 90,4 | 94,1 | 95,0 | 93,2 | 2,0 |
| 25 | 95,5 | 98,2 | 100 | 97,9 | 1,8 |
| 30 | 98,2 | 100 | 100 | 99,4 | 0,8 |
| 35 | 100 | 100 | 100 | 100 | 0 |
| 40 | 100 | 100 | 100 | 100 | 0 |
| 45 | 100 | 100 | 100 | 100 | 0 |
| 60 | 100 | 100 | 100 | 100 | 0 |

Tableau 2

| Temps (minute) | Comprimé n° 1 | Comprimé n° 2 | Comprimé n° 3 | Moyenne (%) | Ecart-type |
|---|---|---|---|---|---|
| 2 | 25,1 | 21,7 | 21,6 | 22,8 | 1,6 |
| 6 | 39,1 | 34,4 | 40,2 | 37,9 | 2,5 |
| 10 | 53,4 | 47,9 | 51,7 | 51,0 | 2,3 |
| 15 | 61,4 | 57,7 | 64,3 | 61,1 | 2,7 |
| 20 | 69,5 | 66,7 | 72,9 | 69,7 | 2,5 |
| 25 | 71,1 | 73,4 | 80,9 | 77,1 | 3,1 |
| 30 | 82,2 | 77,6 | 86,5 | 82,1 | 3,6 |
| 35 | 90,4 | 80,7 | 89,6 | 86,9 | 4,4 |
| 40 | 92,7 | 85,1 | 94,5 | 90,8 | 4,1 |
| 45 | 95,0 | 88,0 | 98,7 | 93,9 | 4,4 |
| 60 | 100 | 93,7 | 100 | 97,9 | 3,0 |

**EXEMPLE 3 : Etude chez le lapin**

L'objectif est de déterminer chez le lapin de manière objective les propriétés irritantes éventuelles et l'effet mydriatique (le moment d'apparition de la mydriase et son évolution dans le temps) provoqué par les comprimés lors de leur administration unique dans l'oeil du lapin qui consiste à placer une de ces formes dans le cul-de-sac conjonctival du lapin.

1. Protocole d'étude

* **Espèce**

Lapin Albinos Néo Zélandais, en provenance d'un élevage spécialisé dont le poids est compris entre 1,5 kg et 2,5 kg.
Les animaux sont identifiés individuellement par tatouage de l'oreille.

* **Stabulation**

Les animaux sont stabulés en cage aux dimensions normalisées. Les cages sont placées dans une animalerie climatisée (17-21° C) maintenue à un degré hygrométrique entre 45 et 65 % d'humidité relative sauf pendant les heures de nettoyage dans laquelle l'air filtré non recyclé est renouvelé. Le cycle nycthéméral est artificiellement recréé dans les conditions suivantes : 12 heures de lumière / 12 heures d'obscurité.

* **Alimentation**

Aliments V.A.R. 112

* **Eau de boisson**

De l'eau de ville est distribuée à volonté dans des biberons en propylène avec tétine en acier inoxydable.

* **Observation des animaux**

Cette observation s'effectue par examen direct. Après la pose du comprimé dans le cul-de-sac conjonctival, les paupières sont closes environ 10 secondes pour éviter le rejet du comprimé. Seulement l'oeil gauche des lapins est traité, l'oeil droit sert de témoin. Lors du traitement, les animaux sont maintenus 1 heure dans une boîte à contention, de façon qu'ils ne puissent se frotter l'oeil puis ils sont remis en cage après retrait du comprimé à l'aide d'une pince. Cependant pour les formules 1 et 2, les animaux ont été maintenus 1 heure supplémentaire dans la boîte de contention après le retrait de la forme, pour une observation plus facile de la mydriase. Mais devant la mauvaise tolérance des lapins au traitement, cette prolongation de 1 heure en boîte de contention n'a pas été conservée pour les autres lots de comprimés.

Les différentes caractéristiques de l'irritation oculaire et les différents stades de la mydriase sont quantifiées par cotation et ici seront appréciées à l'oeil nu.

**Lésions oculaires**

Les différentes caractéristiques et leur cotation décrites ci-après sont celles définies dans la procédure de travail.

**a) Chémosis (gonflement)**

| - Pas de gonflement | G-0 |
|---|---|
| - Gonflement léger, y compris de la membrane nictitante | G-1 |
| - Gonflement avec éversion de la paupière | G-2 |
| - Gonflement avec paupières à demi fermées | G-3 |
| - Gonflement avec paupières fermées plus qu'à moitié ou complètement | G-4 |

**b) Larmoiement**

| - Pas de larmoiement | L-0 |
|---|---|
| - Larmoiement léger | L-1 |
| - Larmoiement avec humidification des paupières et des poils avoisinants les paupières | L-2 |
| - Larmoiement avec humidification des paupières et des poils sur de larges surfaces de l'oeil | L-3 |

**c) Rougissement de la conjonctive palpébrale**

| - Vaisseaux normaux | V-0 |
|---|---|
| - Vaisseaux nettement plus injectés que la normale | V-1 |
| - Vaisseaux difficiles à distinguer individuellement couleur rouge vif diffuse | V-2 |
| couleur rouge foncé | V-3 |

**d) Lésion cornéenne**

Degré d'opacification :

| | |
|---|---|
| - Aucune modification visible ni perte de brillant au soleil | O-0 |
| - Présence de zones translucides (diffuses ou disséminées), détails de l'iris clairement visibles | O-1 |
| - Présence d'une zone translucide facilement identifiable, détails de l'iris légèrement masqués | O-2 |
| - Présence d'une opalescente, aucun détail de l'iris visible, contour de la pupille à peine discernable | O-3 |
| - Présence d'une opacité cornéenne totale rendant l'iris et la pupille invisible | O-4 |

**Stades de la mydriase :**

M-1 :  Début de la mydriase, la pupille est légèrement plus dilatée jpar rapport à l'oeil non traité
M-2 :  Mydriase moyenne
M-3 :  Mydriase maximale

2. Résultats

\* **Aspect galénique**

Les différents états des comprimés observés après ablation sont en accord avec les essais **in vitro** préalablement réalisés. En effet, les comprimés de la formule 1 placés dans un cristallisor avec de l'eau distillée deviennent rapidement friables et **in vivo** lors de leur retrait de l'oeil des lapins, ils se brisent en laissant des débris. Les comprimés de la formule 2 restent entiers et ne sont que ramolli par le contact avec le liquide lacrymal. Par la suite, une meilleure dureté de ces comprimés à été recherchée de façon à pouvoir les retirer plus facilement et sans débris. Ces objectifs ont été atteints avec la formule 5 et la formule 5' qui comporte la présence d'un enrobage.

**ACTIVITE DES COMPRIMES**

\* **Formule 1**

Le dosage de ces comprimés en néosynéphrine est très proche de celui de la D.L. 50 du rat (dose pour laquelle après administration unique la moitié des animaux traités meurent) administrée par voie sous-cutanée. Ces formes ont quand même été administrées aux lapins pour savoir si les principes actifs sont libérés **in vivo** et essayer d'apprécier l'importance de leur activité. On obtient une mydriase flash, qui devient rapidement collatérale, maximale en moins de 10 minutes. Les lapins présentent moins d'1 heure après d'important signes de surdosage et meurent peu après le retrait de ces formes donc moins de 2 heures après leur administration. Il y a donc eu une libération presque immédiate des principes actifs puis passage systémique, comparativement à une voie intraveineuse.

\* **Formule 2**

Bien que dix fois moins dosés en tropicamide, les lapins du lot 2 n'ont pas pu résister à la dose encore importante de néosynéphrine. L'effet mydriatique a été moins violent et un des lapins sur trois à survécu. Aux alentours de la 4ème heure, la mydriase collatérale disparait et la mydriase de l'oeil traité bien que bonne n'est plus maximale. Le dosage est encore trop fort et les lapins présentent de nombreux signes de surdosage.

\* **Formule 4**

Ces comprimés sont beaucoup moins dosés en néosynéphrine dont le dosage est réduit de plus de 50 %. La tolérance est nettement améliorée car les lapins ne présentent pas d'effets indésirables visibles. L'effet mydriatique beaucoup plus progressif n'est plus collatéral. La mydriase passe par deux stades intermédiaires (M-1 et M-2) avant d'être maximale (environ 25 minutes après la pose du comprimé). L'effet mydriatique est stable durant au moins 5 heures.

**\* Formule 5 et 5'**

Pour ces comprimés la cinétique de l'effet mydriatique est encore plus ralentie : la mydriase maximale est obtenue peu avant 30 minutes. La tolérance est excellente après administration des comprimés enrobés de la formule 5' pour lesquels les lapins ne semblent nullement affectés par le traitement. L'enrobage jouerait le rôle de matrice qui freinerait la libération de la néosynéphrine et de la tropicamide. Ces observations sont correlées par la cinétique **in vitro** décrite précédemment. La mydriase reste bonne au moins 4 heures après administration de ces comprimés.

**\* Lésions oculaires**

Les comprimés du lot 1 de par leur taille et leur consistance provoquent de nombreuses irritations notamment des larmoiements importants. Les vaisseaux de la conjonctive palpébrale sont légèrement plus injectés que la normale et les paupières sont un peu gonflées. Après le retrait des comprimés ces signes disparaissent, ils semblent en majeur partie dus à la présence de ces comprimés dans le cul-de-sac conjonctival.

Les comprimés des autres formules ne comportent que de légers larmoiements en général. Les lapins qui ont eu les comprimés enrobés ne présentent aucun signe d'irritation. Cette bonne tolérance serait due à la petite taille des comprimés, leur dureté et leur aspect plus lisse. De plus, les principes actifs semblent libérés beaucoup plus progressivement que pour les autres comprimés des précédentes formules.

**EXEMPLE 4 : Etude chez l'homme**

Les essais sont effectués sous surveillance médicale par un clinicien suivant un protocole établi.

1. Protocole d'étude

L'étude a eu lieu sur 8 sujets, 4 ont reçu les comprimés de la formule 5, les 4 autres ceux de la formule 5'. A chaque patient n'a été administré qu'un seul comprimé, le deuxième oeil non traité a servi de témoin.

Les observations cliniques rapportées par le médecin (état de la mydriase, réactions locales et effet secondaires éventuels) ainsi que l'état du comprimé après élimination sont décrits dans le tableau A pour les comprimés de la formule 5. Les observations détaillées pour chaque sujet après administration des comprimés de la formule 5' n'ont pas été communiquées les résultats s'étant révélés identiques dans les quatre cas. Le clinicien a donc donné une description de l'état général des 4 sujets.

A chaque patient est attribué le numéro 1, 2, 3 ou 4 .

2- Résultats

**Résultats obtenus après administration des comprimés de la formule 5 :**

Les observations cliniques sont décrites dans le tableau A ci-dessous.

Tableau A

| Obs.\* n° | Etat de la mydriase | Réaction locale | Effets secondaires |
|---|---|---|---|
| 1 | excellente | néant | aucun |
| 2 | valable | pétéchies hémoragiques | aucun |
| 3 | excellente | sillon très marqué, pétéchies | aucun |
| 4 | excellente | néant | aucun |

\*Obs = Observation

Pour les quatre sujets, du fait de la surface relativement lisse des comprimés, ils glissent facilement dans le cul-de-sac conjonctival. Cependant, les bords des comprimés sont acérés, cela explique, en partie, les pétéchies hémorragiques. La taille des comprimés est jugée idéale. Après 30 minutes, le comprimé est très ramolli, il reste toutefois entier lors de l'ablation qui s'effectue presque spontanément en appuyant simplement sur le bord de la paupière inférieure. Les doses du produit mydriatiques semblent bonnes.

**Résultats obtenus après administration des comprimés de la formule 5' :**

Les résultats énumérés ci-dessous sont identiques pour les 4 sujets.

- La mydriase est excellente après 20 minutes,
- Empreinte au niveau du site conjonctival, avec quelques pétéchies hémoragiques très fines. Pas de réaction oedémateuse,
- Aucune réaction neuro-végétative générale n'a été signalée
- L'enrobage paraît donc efficace, en effet il n'y a pas débris.

**Revendications**

1. Forme galénique destinée à l'administration oculaire, caractérisée en ce qu'il s'agit d'un comprimé solide insoluble qui comporte, de l'intérieur vers l'extérieur :

   - une matrice, constituée d'au moins un polymère, et renfermant au moins un principe actif,
   - une première couche de glycérides.

2. Comprimé selon la revendication 1, caractérisé en ce qu'il comporte en outre une seconde couche, formant une membrane externe semi-perméable.

3. Comprimé selon l'une des revendications 1 ou 2, caractérisé en ce que le polymère utilisé pour la matrice est choisi dans le groupe comprenant les polymères suivants : polyéthylène, polymère acrylique, chlorure de polyvinyle, dérivés cellulosiques du type HPMC, alcool polyvinylique, copolymères d'acide acrylique et méthacrylique (Eudragit ®), ou leurs mélanges.

4. Comprimé selon l'une des revendications 1 à 3, caractérisé en ce que les glycérides sont des glycérides solides, naturels ou semi-synthétiques.

5. Comprimé selon la revendication 4, caractérisé en ce que le glycéride est du behénate de glycérol (ou Compritol ®).

6. Comprimé selon l'une des revendications 2 à 5, caractérisé en ce que la membrane semi-perméable est formée d'un dérivé thermoplastique de cellulose, par exemple d'éthylcellulose.

7. Comprimé selon l'une des revendications 1 à 6, caractérisé en ce que le principe actif est choisi dans le groupe comprenant les mydriatiques, les antibiotiques et les antiinflammatoires.

8. Comprimé selon l'une des revendications 1 à 7, caractérisé en ce que le principe actif est présent à un taux compris entre 1 et 60%, le polymère de la matrice est présent à un taux compris entre 25 et 70%, et les glycérides sont présents à un taux de 8 à 40% par rapport au poids total du comprimé.

9. Comprimé selon l'une des revendications 1 à 8, caractérisé en ce qu'il a une longueur comprise entre environ 2,5 et 4 mm et un diamètre compris entre environ 1 et 3 mm.

10. Procédé de préparation d'un comprimé insoluble selon l'une des revendications 1 à 9, caractérisé en ce qu'on effectue les étapes suivantes:

    a) mélange du ou des principes actifs avec le ou les polymères constitutifs de la matrice,
    b) de manière facultative, granulation du mélange obtenu après mouillage et incorporation d'adjuvants,
    c) addition des glycérides et mélange,
    d) compression directe du mélange obtenu à l'issue de l'étape précédente.

11. Procédé selon la revendication 10 de préparation d'un comprimé selon l'une des revendications 2 à 9, caractérisé en ce que après l'étape d), on effectue une étape d'enrobage du comprimé par un dérivé thermoplastique en solution dans un solvant organique.

12. Procédé selon la revendication 11, caractérisé en ce que l'enrobage est effectué par trempage des comprimés dans une solution d'éthylcellulose dans un solvant organique, puis séchage à l'étuve.

**13.** Utilisation d'une matrice polymère renfermant au moins un principe actif et revêtue d'une couche de glycérides, pour la fabrication d'un comprimé solide insoluble, destiné à l'administration oculaire, et apte à être facilement retiré de l'oeil après relargage du ou desdits principes actifs.

**Claims**

**1.** Pharmaceutical dosage form intended for ocular administration, characterized in that it is an insoluble solid tablet which possesses, from the inside to the outside:

- a matrix comprising at least one polymer and containing at least one active principle,
- a first layer of glycerides.

**2.** Tablet according to Claim 1, characterized in that it possesses, in addition, a second layer, forming a semipermeable outer membrane.

**3.** Tablet according to either of Claims 1 and 2, characterized in that the polymer used for the matrix is chosen from the group comprising the following polymers: polyethylene, acrylic polymer, polyvinyl chloride, cellulose derivatives of the HPMC type, polyvinyl alcohol, acrylic and methacrylic acid copolymers (Eudragit®) or mixtures thereof.

**4.** Tablet according to one of Claims 1 to 3, characterized in that the glycerides are natural or semisynthetic solid glycerides.

**5.** Tablet according to Claim 4, characterized in that the glyceride is glyceryl behenate (or Compritol®).

**6.** Tablet according to one of Claims 2 to 5, characterized in that the semipermeable membrane is formed from a thermoplastic derivative of cellulose, for example ethylcellulose.

**7.** Tablet according to one of Claims 1 to 6, characterized in that the active principle is chosen from the group comprising mydriatics, antibiotics and antiinflammatories.

**8.** Tablet according to one of Claims 1 to 7, characterized in that the active principle is present at a level of between 1 and 60%, the polymer of the matrix is present at a level of between 25 and 70%, and the glycerides are present at a level of 8 to 40%, relative to the total weight of the tablet.

**9.** Tablet according to one of Claims 1 to 8, characterized in that it is between approximately 2.5 and 4 mm in length and between approximately 1 and 3 mm in diameter.

**10.** Process for preparing an insoluble tablet according to one of Claims 1 to 9, characterized in that the following steps are performed:

a) mixing of the active principle or principles with the polymer or polymers constituting the matrix,
b) optionally, granulation of the mixture obtained after wetting and incorporation of adjuvants,
c) addition of the glycerides and mixing,
d) direct tabletting of the mixture obtained at the end of the preceding step.

**11.** Process according to Claim 10 for preparing a tablet according to one of Claims 2 to 9, characterized in that, after step d), a step of coating the tablet with a thermoplastic derivative dissolved in an organic solvent is performed.

**12.** Process according to Claim 11, characterized in that the coating is performed by dipping the tablets in a solution of ethylcellulose in an organic solvent, followed by drying in an oven.

**13.** Use of a polymer matrix containing at least one active principle and coated with a layer of glycerides, for the manufacture of an insoluble solid tablet intended for ocular administration and capable of being easily removed from the eye after release of the said active principle or principles.

**Patentansprüche**

**1.** Galenische Form für die okuläre Verabreichung, dadurch gekennzeichnet, daß es sich dabei um eine unlösliche feste Tablette handelt, die von außen nach innen enthält:

- eine Matrix, bestehend aus mindestens einem Polymer, die mindestens einen Wirkstoff (aktives Prinzip) enthält, und
- eine erste Glycerid-Schicht.

2. Tablette nach Anspruch 1, dadurch gekennzeichnet, daß sie außerdem eine zweite Schicht enthält, die eine semipermeable äußere Membran bildet.

3. Tablette nach einem der Ansprüche 1 oder 2, dadurch gekennzeichnet, daß das für die Matrix verwendete Polymer ausgewählt wird aus der Gruppe, welche die folgenden Polymeren umfaßt: Polyethylen, Acrylpolymer, Polyvinylchlorid, Cellulosederivate vom HPMC-Typ, Polyvinylalkohol, Acrylsäure/Methacrylsäure-Copolymere (Eudragit®) oder ihre Mischungen.

4. Tablette nach einem der Ansprüche 1 bis 3, dadurch gekennzeichnet, daß es sich bei den Glyceriden um feste natürliche oder halbsynthetische Glyceride handelt.

5. Tablette nach Anspruch 4, dadurch gekennzeichnet, daß es sich bei dem Glycerid um Glycerinbehenat (oder Compritol®)handelt.

6. Tablette nach einem der Ansprüche 2 bis 5, dadurch gekennzeichnet, daß die semipermeable Membran aus einem thermoplastischen Cellulosederivat, beispielsweise aus Ethylcellulose, gebildet worden ist.

7. Tablette nach einem der Ansprüche 1 bis 6, dadurch gekennzeichnet, daß der Wirkstoff (das aktive Prinzip) ausgewählt wird aus der Gruppe, welche die Mydriatika, die Antibiotika und die entzündungshemmenden Mittel umfaßt.

8. Tablette nach einem der Ansprüche 1 bis 7, dadurch gekennzeichnet, daß der Wirkstoff (das aktive Prinzip) in einem Gehalt zwischen 1 und 60 % vorliegt, daß das Polymer der Matrix in einem Gehalt zwischen 25 und 70 % vorliegt und daß die Glyceride in einem Gehalt von 8 bis 40 %, bezogen auf das Gesamtgewicht der Tablette, vorliegt.

9. Tablette nach einem der Ansprüche 1 bis 8, dadurch gekennzeichnet, daß sie eine Länge zwischen etwa 2,5 und 4 mm und einen Durchmesser zwischen etwa 1 und 3 mm hat.

10. Verfahren zur Herstellung einer unlöslichen Tablette nach einem der Ansprüche 1 bis 9, dadurch gekennzeichnet, daß man die folgenden Stufen durchführt:

    a) Mischen des oder der Wirkstoffe (aktiven Prinzipien) mit dem oder den die Matrix aufbauenden Polymeren,
    b) gegebenenfalls Granulieren der nach dem Anfeuchten und nach Einarbeitung von Adjuvantien erhaltenen Mischung,
    c) Zugabe von Glyceriden und Mischen und
    d) direktes Pressen der am Ende der vorhergehenden Stufe erhaltenen Mischung.

11. Verfahren nach Anspruch 10 zur Herstellung einer Tablette nach einem der Ansprüche 2 bis 9, dadurch gekennzeichnet, daß man nach der Stufe (d) die Tablette mit einem thermoplastischen Derivat, gelöst in einem organischen Lösungsmittel, umhüllt.

12. Verfahren nach Anspruch 11, dadurch gekennzeichnet, daß die Umhüllung durchgeführt wird durch Eintauchen der Tabletten in eine Lösung von Ethylcellulose in einem organischen Lösungsmittel und anschließendes Trocknen im Trockenschrank.

13. Verwendung einer Polymermatrix, die mindestens einen Wirkstoff (aktives Prinzip) enthält und von einer Glyceridschicht bedeckt ist, zur Herstellung einer unlöslichen festen Tablette für die okuläre Verabreichung, die nach dem Aussalzen des oder der Wirkstoffe (aktiven Prinzipien) leicht wieder aus dem Auge entfernt werden kann.

Figure 1

comprimés du lot 5